# EUROPEAN PATENT SPECIFICATION

(11) **EP 3 122 257 B1**
(45) Date of publication and mention of the grant of the patent: **17.02.2021**
(21) Application number: 14724791.0
(22) Date of filing: 27.03.2014
(51) Int. Cl.: A61B 8/00, G06F 3/041

(54) **ULTRASOUND IMAGING SYSTEM TOUCHSCREEN USER INTERFACE**
BENUTZERSCHNITTSTELLE FÜR BERÜHRUNGSBILDSCHIRM EINES ULTRASCHALLBILDGEBUNGSSYSTEMS
INTERFACE UTILISATEUR À ÉCRAN TACTILE DE SYSTÈME D'ÉCHOGRAPHIE

(43) Date of publication of application: 01.02.2017
(73) Proprietor: B-K Medical ApS, 2730 Herlev (DK)
(72) Inventor: NIELSEN, Torben, DK-2300 Copenhagen S (DK); DUNKAN, Kaj, DK-4295 Stenlille (DK); HANSEN, Jesper, DK-2400 Kobenhavn NV (DK); ANTOL, John, Nahant, Massachusetts 01908 (US); LEISMANN, Michael, DK-2650 Hvidovre (DK)
(74) Representative: Gevers Patents
(86) International application number: PCT/IB2014/060237
(87) International publication number: WO 2015/145213

(56) References cited:
- US-A1- 2012 289 828
- US-A1- 2013 261 449
- US-A1- 2014 204 699

## Description

### TECHNICAL FIELD

The following generally relates to an ultrasound imaging system and more particularly to an ultrasound imaging system touchscreen user interface.

### BACKGROUND

Ultrasound (US) imaging provides useful information about the interior characteristics (e.g., anatomical tissue, material flow, etc.) of a subject under examination. An ultrasound imaging system has included a probe with an ultrasound transducer array, a console, a display and a keyboard. The transducer array transmits an ultrasound signal into a field of view and receives echoes produced in response to the signal interacting with structure therein. The echoes are processed by the console, which generates images indicative of the structure that are visually presented in the display region.

An example suitable keyboard has a coherent, flat surface, without any holes, e.g. glass, combined with a touch screen and e.g. a TFT panel. Unfortunately, such a keyboard is not well-suited for navigation on the surface of the keyboard by the user's hand to locate and use a control of interest of the keyboard without the user having to look at the keyboard. Exacerbating the problem, some controls will have more than one function or mode. Furthermore, such a keyboard is not well-suited making measurements, etc. with high precision.

US 2012/289828 A1 and US 2013/261449 A1 each disclose an ultrasound imaging system according to the preamble of claims 1 and 5.

### SUMMARY

Aspects of the application address the above matters, and others.

In one aspect, an ultrasound imaging system includes a probe with a transducer array with at least one transducer element. The ultrasound imaging system further includes a console with a controller, which controls the at least one transducer element, and an echo processor. The ultrasound imaging system further includes a display monitor. The ultrasound imaging system further includes a touch screen user interface, including: a touch panel with a first major surface and a first recess in the first major surface; and at least one touch sensitive control disposed in the recess. The first recess, comprises: a touch sensitive flat, planar surface configured for sensing a first gesture thereon which activates the touch sensitive flat, planar surface, and for sensing a subsequent gesture thereon that invokes a predetermined action of the at least one touch sensitive control.

In another aspect, an ultrasound imaging system includes a probe with a transducer array with at least one transducer element. The ultrasound imaging system further includes a console with a controller, which controls the at least one transducer element, and an echo processor. The ultrasound imaging system further includes a display monitor. The ultrasound imaging system further includes a touch screen user interface, including: a touch panel with a first major surface and a first recess in the first major surface; and at least one touch sensitive control disposed in the recess. The first recess, comprises: a second recess disposed therein, and a touch sensitive flat, actuating planar surface, the second recess, comprising a touch sensitive flat, active planar surface, wherein the touch sensitive flat, actuating planar surface senses a first gesture thereon, which activates the touch sensitive flat, active planar surface.

Those skilled in the art will recognize still other aspects of the present application upon reading and understanding the attached description.

### BRIEF DESCRIPTION OF THE DRAWINGS

The application is illustrated by way of example and not limitation in the figures of the accompanying drawings, in which like references indicate similar elements and in which:
Figure 1 schematically illustrates an example imaging system with a touchscreen control user interface;
Figure 2 schematically illustrates an example touch panel of the touchscreen control user interface;
Figure 3 schematically illustrates a top down view of an example trackball touch control of an active region of the panel of the touchscreen control user interface;
Figure 4 schematically illustrates a cross-sectional view of the example trackball touch control of Figure 3;
Figure 5 schematically illustrates a perspective view of the example trackball touch control of Figure 3;
Figures 6-9 illustrates example operation of the example trackball touch control of Figure 4;
Figures 10-13 illustrates variations of the example trackball touch control of Figures 4 and 14;
Figure 14 schematically illustrates a top down view of an example toggle touch control of the active region of the panel of the touchscreen control user interface;
Figure 15 schematically illustrates a cross-sectional view of the example toggle touch control of Figure 14;
Figure 16 schematically illustrates a perspective view of the example toggle touch control of Figure 14;
Figures 17-20 illustrates example operation of the example toggle touch control of Figure 14; and
Figure 21 illustrates an example method in accordance with the embodiments disclosed herein.

### DETAILED DESCRIPTION

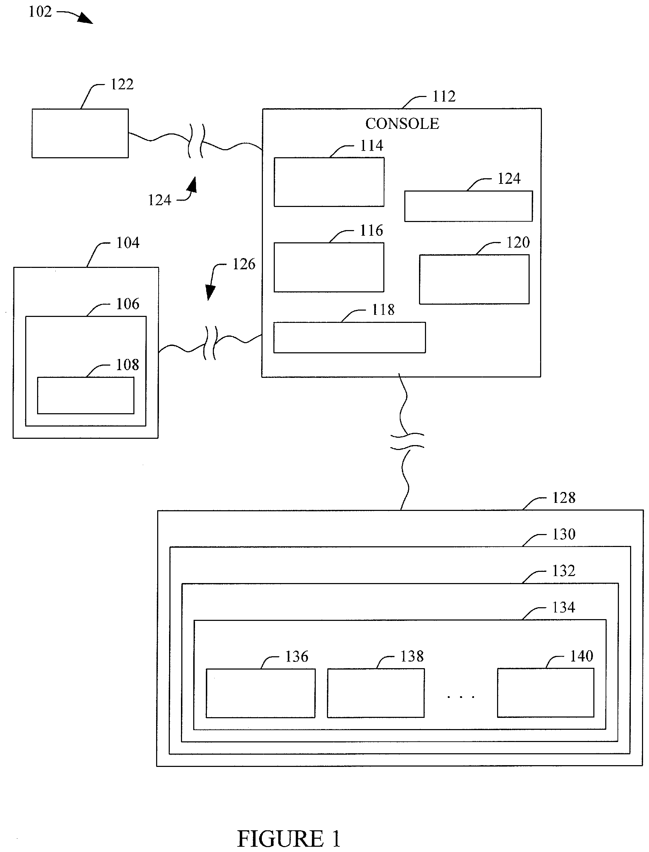

Figure 1 schematically illustrates an ultrasound (US) imaging system 102. The ultrasound imaging system 102 includes a probe 104 with a one-dimensional (ID) or two-dimensional (2D) transducer array 106 with at least one transducer element 108. The at least one transducer element 108 is configured to transmit ultrasound signals and receive echo signals. Suitable array configurations include, but are not limited to, linear, curved (e.g., concave, convex, etc.), circular, etc., full populated or sparse, etc.

The ultrasound imaging system 102 further includes a console 112. The console 112 includes transmit circuitry 114 that selectively excites one or more of the at least one transducer element 108. More particularly, the transmit circuitry 114 generates a set of pulses (or a pulsed signal) that are conveyed to the transducer array 106. The set of pulses excites the at least one transducer element 108, causing the at least one transducer element 108 to transmit an ultrasound signal into an examination scan field of view.

The console 112 further includes receive circuitry 116 that receives a set of echoes (or echo signals) generated in response to the transmitted ultrasound signals. The echoes, generally, are a result of the interaction between the emitted ultrasound signals and the object (e.g., flowing blood cells, organ cells, etc.) in the scan field of view. The receive circuit 116 may be configured for spatial compounding, filtering (e.g., FIR and/or IIR), and/or other echo processing.

The console 112 further includes an echo processor (e.g., a beamformer) 118 that processes the received echoes. For example, in B-mode, this may include applying time delays and weights to the echoes and summing the delayed and weighted echoes, and generating an image. The console 112 further includes a scan converter 120 that scan converts the processed data for display, e.g., by converting the beamformed data to the coordinate system of a display monitor used to visually present the processed data.

The console 112 further includes a controller 124 that controls the various components of the system 102. For example, such control may include controlling the transmit circuitry 114 to excite individual or groups of the at least one transducer element 108 for an A-mode, B-mode, C-plane, and/or other data acquisition mode, steering and/or focusing the transmitted signal, etc., actuating the at least one transducer element 108 for steering and/or focusing the received echoes, etc.

The ultrasound imaging system 102 further includes a display monitor 122. The display monitor 122 can be a cathode ray tube (CRT), a liquid crystal display (LCD), a light emitting diode (LED), and/or other display monitor. The display monitor 122 includes a display region, which can visually present images and/or flow information generated by the console 112.

The system 102 further includes a touch screen user interface 128 with a touch panel 130. The touch panel 130 includes a resistive, a capacitive, an acoustic, an infrared, an optical, a piezoelectric, and/or other region. The touch panel 130 includes an active region(s) 132 with a touch sensitive control(s) 134. A touch sensitive control(s) 134 is actuated by a gesture (e.g., a press, a swipe, a touch, etc.) on the touch sensitive control(s) 134 with one or more fingers, a stylus, a glove, etc.

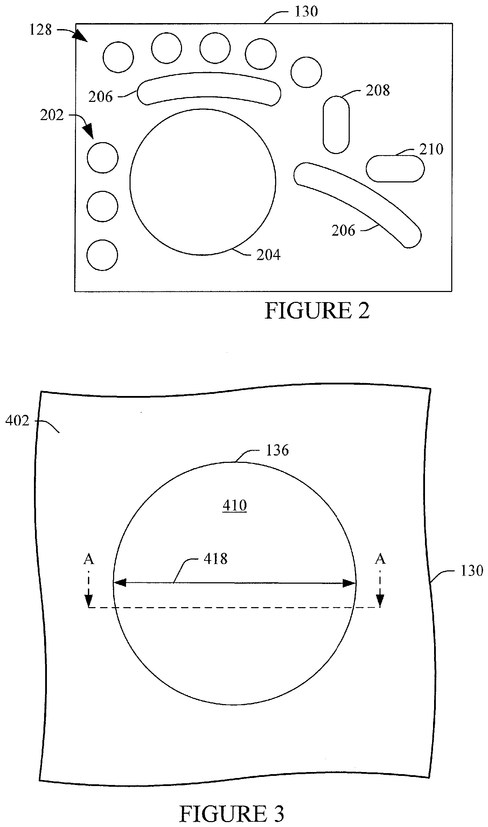

Examples of the touch sensitive control(s) 134 include a trackball control 136 for navigating a graphical pointer displayed in the display region of the display monitor 122, a toggle control 138 for incrementing and decrementing value corresponding to focus, depth, zoom, etc. and/or other control(s) 140. Briefly turning to Figure 2, an example of the touch screen user interface 128 with a plurality of the touch sensitive control(s) 134 is illustrated.

In Figure 2, the touch sensitive control(s) 134 include circular shaped controls 202 and 204. The controls 202 have a first size (i.e., diameter), and the control 204 has a second size, which is larger than the first size. In a variation, the touch sensitive control(s) 134 include only a single size circular control or more than two sizes of circular controls. Furthermore, the illustrated number of the circular shaped controls 202 and 204 is not limiting; in a variation, there could be more or less of the circular controls 202 and 204.

The touch sensitive control(s) 134 also include rectangular shaped controls 206 in which the rectangular shaped controls 206 include curved sides and rounded corners. In this example, the rectangular shaped controls 206 are the same size. However, in a variation, the touch sensitive control(s) 134 include multiple different size rectangular shaped controls 206. Furthermore, the illustrated number of the rectangular shaped controls 206 is not limiting; in a variation, there could be more or less of the circular controls 202 and 204.

The touch sensitive control(s) 134 also include oval or elliptical shaped controls 208 and 210. In this example, the elliptical shaped controls 208 and 210 are the same size. However, in a variation, the touch sensitive control(s) 134 include multiple different size elliptical shaped controls 208 and 210. Furthermore, the illustrated number of the elliptical shaped controls 208 and 210 is not limiting; in a variation, there could be more or less of the elliptical shaped controls 208 and 210.

Returning to Figure 1, the touch panel 130 may also include one or more non-active regions, a display such as a LCD, a thin film transistor (TFT) LCD, an organic light-emitting diode (OLED) and/or other display, and/or other features. Another example of a suitable interface is described in US patent application serial number 13/748,653, which was filed on January 24, 2013, and entitled "Ultrasound Imaging System,".

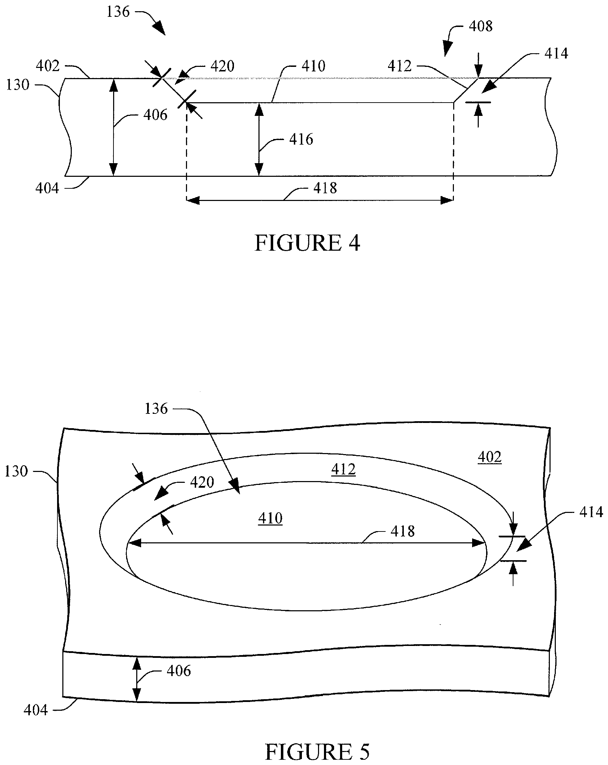

Figures 3, 4 and 5 illustrate an example of the trackball control 136 (Figure 1) implemented using the larger circular shaped control 204 (Figure 2). Figure 3 shows a top down view of the trackball control 136 in connection with a sub-portion of the touch panel 130. Figure 4 shows a cross-sectional view of the trackball control 136 along lines A-A. Figure 5 shows a perspective view of the trackball control 136.

With reference to figures 3, 4 and 5, the trackball control 136 has a first major surface 402 and a second major surface 404, which is parallel to and opposite from the first major surface 402. The first and second major surfaces 402 and 404 are separated by a material of the touch panel 130. In the illustrated embodiment, the first and second major surfaces 402 and 404 are separated are separated by a non-zero distance 406, which is in a range of 1 to 6 millimeters (mm).

The first major surface 402 includes a recess 408. The recess has a generally flat, planar active surface 410 and a side wall 412, which extends from the first major surface 402 to the generally flat, planar active surface 410 within the touch panel 130. The generally flat, planar surface 410 is offset from the first major surface 402 by a non-zero distance 414, which is in a range of 0.1 to 1 mm.

The recess 408 has a diameter 418, which is in a range of 40 to 100 mm. The illustrated side wall 412 extends linearly or non-linearly from the first major surface 402 to the recess 408. The side wall 412 has a length 420, which is in a range of 0.1 to 4 mm. It is to be appreciated that the distances 406, 414, 416, 418, and 420 are provided for explanatory purposes and are not limiting. Other ranges for these distances are contemplated herein.

The circular trackball control 136 in Figure 3-5 resembles a trackball and thereby is easily recognized by ultrasound users. Configured to provide trackball functionality, the circular trackball control 136 can be used to control a cursor on the ultrasound image with higher accuracy compared to managing the ultrasound image with direct touch input. Using the circular trackball control 136 as such, the circular trackball control 136 can be used to make measurements, etc. with high precision.

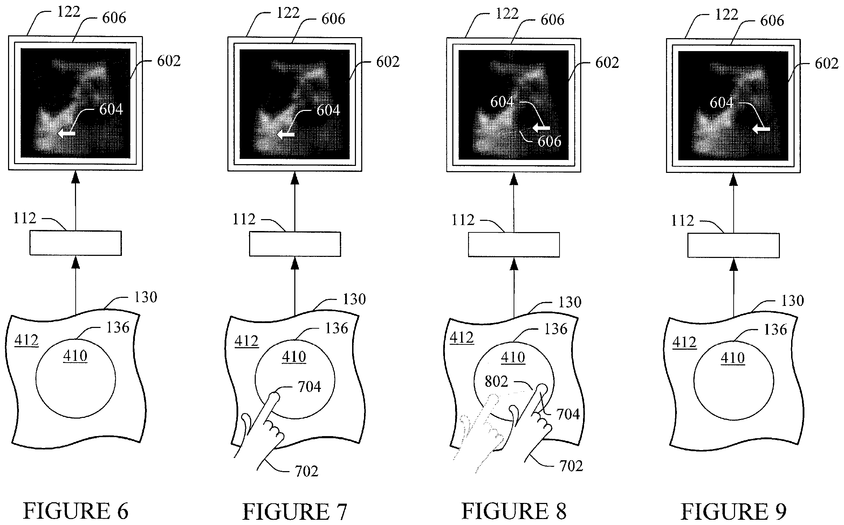

Figures 6, 7, 8 and 9 show example operation of the trackball control 136 of Figures 3, 4 and 5. Figure 6 shows the sub-portion of the touch panel 130 with the trackball control 136 in connection with the console 112 and the display 122, which displays an image 602 and displays a graphical cursor 604 (e.g., an arrow in the illustrated embodiment) in a display region 606 of the display 122.

In Figure 7, a user 702 touches, with a finger 704, the generally flat, planar active surface 410. In response thereto, the touch screen user interface 128 conveys a signal to the console 112. The signal indicates a current location of the touch on the generally flat, planar active surface 410 of the trackball control 136. The controller maps the current finger position to a current location of the graphical cursor 604 on the image 602.

In Figure 8, the user 702 slides the finger 704 across the generally flat, planar active surface 410. As the finger slides, the touch screen user interface 128 conveys a signal to the console 112. The signal indicates each new current location of the touch on the generally flat, planar active surface 410 of the trackball control 136. The controller 124 moves the graphical cursor 604 along a path 606 that corresponds to the movement of the finger cross the generally flat, planar active surface 410.

In Figure 9, the user 702 removes the finger 704 from the generally flat, planar active surface 410. Figure 9 shows the sub-portion of the touch panel 130 with the trackball control 136 in connection with the console 112 and the display 122, which displays the image 602 and the graphical cursor 604, at the new location, in the display region 606 of the display 122.

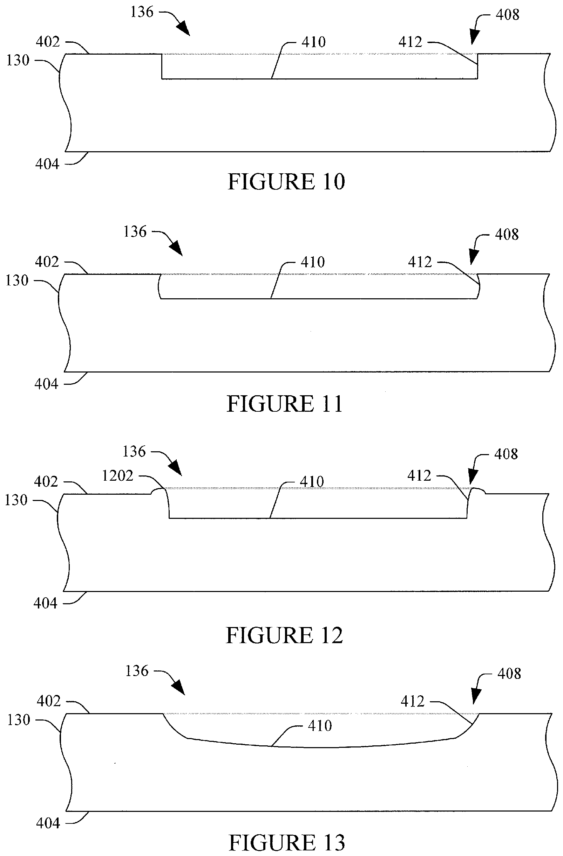

Figures 10, 11, 12 and 13 show variations of the trackball control 136 of Figures 3, 4 and 5. In Figure 10, the side wall 412 extends generally perpendicular between the first major surface 402 and the generally flat, planar active surface 410. In Figure 10, the side wall 412 is a curved surface. In Figure 12, the first major surface 402 and the side wall 412 meet at a region 1202 that is raised above first major surface 402. In Figure 13, the side wall 412 is part of the first major surface 402, which includes a curved active region.

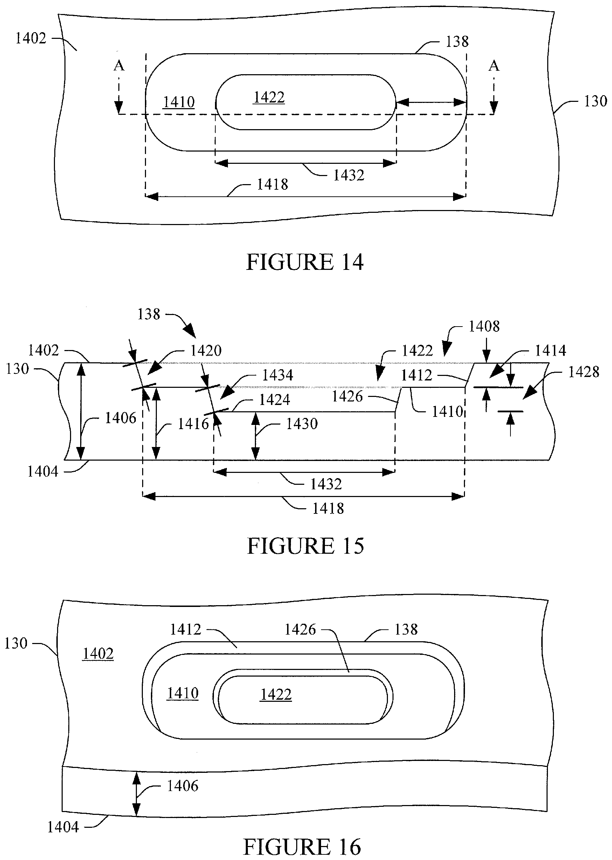

Figures 14, 15 and 16 illustrate an example of the toggle control 138 (Figure 1) implemented using the elliptical shaped control 208 and 210 (Figure 2). Figure 14 shows a top down view of the toggle control 138 in connection with a sub-portion of the touch panel 130. Figure 15 shows a cross-sectional view of the toggle control 138 along lines A-A. Figure 16 shows a perspective view of the toggle control 138.

The toggle control 138 has a first major surface 1402 and a second major surface 1404, which is parallel to and opposite from the first major surface 1402. The first and second major surfaces 1402 and 1404 are separated by a material of the touch panel 130. In the illustrated embodiment, the first and second major surfaces 1402 and 1404 are separated are separated by a first non-zero distance 1406, which is in a range of 1 to 6 mm.

The first major surface 1402 includes a first elliptical recess 1408. The first elliptical recess 1408 has a generally flat, planar active surface 1410 and a first side wall 1412, which extends from the first major surface 1402 to the generally flat, planar active surface 1410 within the touch panel 130. The generally flat, planar surface 1410 is offset from the first major surface 1402 by a second non-zero distance 1414, which is in a range of 0.1 to 0.5 mm and from the second major surface 1404 by a first non-zero distance 1416.

The first elliptical recess 1408 has a first long axis 1418, which is in a range of 20 to 40 mm. The illustrated first side wall 1412 extends linearly from the first major surface 1402 to the first generally flat, planar surface 1410. The first side wall 1412 has a first length 1420, which is in a range of 0.1 to 2 mm. It is to be appreciated that the distances 1406, 1414, 1416, 1418, and 1420 are provided for explanatory purposes and are not limiting. Other ranges for these distances are contemplated herein.

The first elliptical recess 1408 includes a second elliptical recess 1422. The second elliptical recess 1422 has a generally flat, planar actuating surface 1424 and a second side wall 1426, which extends from the generally flat, planar surface 1410 to the generally flat, planar actuating surface 1424 within the touch panel 130. The generally flat, planar actuating surface 1424 is offset from the generally flat, planar active surface 1410 by a third non-zero distance 1428, which is in a range of 0.1 to 0.5 mm, and from the second major surface 1404 by a fourth non-zero distance 1430.

The second elliptical recess 1422 has a second long axis 1432, which is in a range of 2 to 30 mm. The illustrated second side wall 1426 extends linearly or non-linearly from the generally flat, planar active surface 1410 to the generally flat, planar actuating surface 1424. The second side wall 1426 has a second length 1434, which is in a range of 0.1 to 2 mm. It is to be appreciated that the distances 1428, 1430, 1432 and 1434, are provided for explanatory purposes and are not limiting. Other ranges for these distances are contemplated herein.

In a variation, at least one of the first or the second recesses 1408 or 1422 is circular, rectangular, square, and/or otherwise shaped.

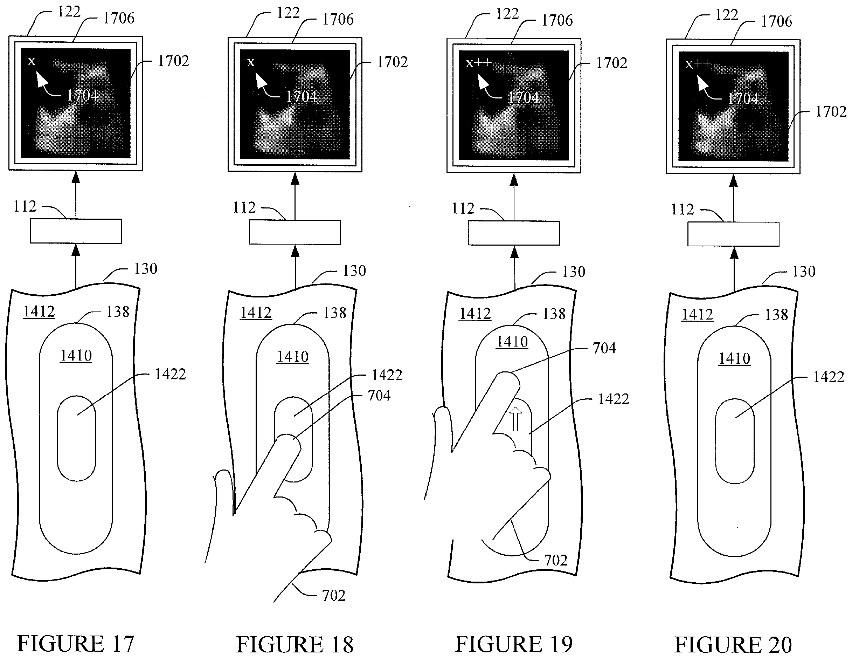

Figures 17, 18, 19 and 20 show example operation of the toggle control 138 of Figures 14, 15 and 16. Figure 17 shows the sub-portion of the touch panel 130 with the toggle control 138 in connection with the console 112 and the display 122, which displays an image 1702 and a graphical indicia 1704 representing a numerical value in a display region 1706 of the display 122.

In Figure 18, the user 702 touches, with the finger 704, the generally flat, planar actuating surface 1422. In response thereto, the touch screen user interface 128 generates a control activation signal, which activates the generally flat, planar active surface 1410.

In Figure 19, the user 702 slides the finger 704 from the generally flat, planar actuating surface 1422 to the generally flat, planar active surface 1410. In response to the finger 704 being on the generally flat, planar active surface 1410, the controller increments the value 1704. In one instance, the controller increments the value 1704 again after a predetermined time delay from the previous increment where the finger 704 remains on the generally flat, planar active surface 1410.

In another instance, the controller increments the value 1704 again after a predetermined time delay from the previous increment in response to the user 702 removing the finger 704 and then touching the generally flat, planar active surface 1410 again. In another instance, a combination and/or other gesture is used to increment the value 1704 again. In the illustrated embodiment, the value 1704 is incremented twice as indicated by the two pluses ("++").

In Figure 20, the user 702 removes the finger 704 from the toggle control 138. In response to a predetermined time delay from the removal of the finger 704, the controller deactivates the generally flat, planar actuating surface 1422.

To decrement the value 1704, the user 702 performs the above, but in the opposite direction as that shown in the Figures 17-20. That is, in this embodiment, moving the finger 704 up the long axis increments the value 1704 and moving the finger 704 down the long axis decrements the value 1704. In a variation, moving the finger 704 up the long axis decrements the value 1704 and moving the finger 704 down the long axis increments the value 1704.

In the illustrated embodiment, moving the finger 704 perpendicular to the long axis does not change the value 1704. In a variation, moving the finger 704 as described above increments and decrements the value 1704 in accordance with a first predetermined value (e.g., 1), and moving the finger 704 perpendicular to the long axis increments and decrements the value 1704 in accordance with a second different predetermined value (e.g., 5).

Similar to the trackball control 136, the transition between the first major surface 1402 to the generally flat, planar active surface 410 and/or between the generally flat, planar active surface 410 and the generally flat, planar actuating surface 422 can be as shown in Figure 10, 11, 12 and 13 and/or otherwise.

Generally, the elliptical toggle control 138 of Figure 14-16 includes a double indentation, which provides a haptic input, thereby helping a user operate the elliptical toggle control 138 without looking at the touch panel 130. The lower level of the indentation is used to activate the upper level, which is used to manipulate the control. Where the elliptical toggle control 138 is configured as a focus bar, the lower level is used to activate the upper level and to identify whether to increase or decrease the focus bar, and the upper level increases or decreases the size in response to the appropriate gesture.

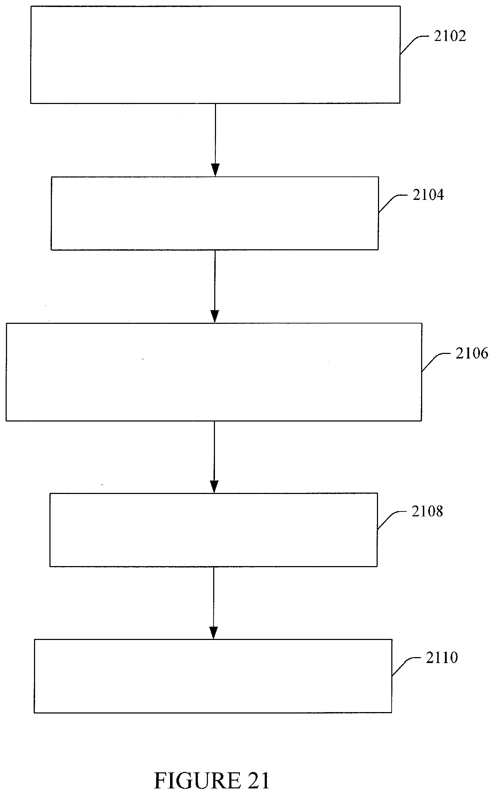

Figure 21 illustrates a method in accordance with the embodiments disclosed herein.

It is to be appreciated that the order of the following acts is provided for explanatory purposes and is not limiting. As such, one or more of the following acts may occur in a different order. Furthermore, one or more of the following acts may be omitted and/or one or more additional acts may be added.

At 2102, a first physical contact with a touch control recessed in a surface of a touch screen user interface is sensed.

At 2104, a first signal indicative of the first physical contact is generated.

At 2106, a second different physical contact with the touch control recessed in the surface of the touch screen user interface is sensed; and

At 2108, a second signal indicative of the second physical contact is generated.

At 2110, a predetermined action is performed based on the first and second physical contact.

The application has been described with reference to various embodiments. Modifications and alterations will occur to others upon reading the application. It is intended that the invention be construed as including all such modifications and alterations, including insofar as they come within the scope of the appended claims.

## Claims

1. An ultrasound imaging system (102), comprising:
a probe (104), including: a transducer array (106) with at least one transducer element (108);
a console (112), including: a controller (124) configured for controlling the at least one transducer element; and an echo processor;
a display monitor (122); and
a touch screen user interface (128), including: a touch panel (130) with a first major surface (402, 1402) and a first recess (408, 1408) in the first major surface; and at least one touch sensitive control (134) disposed in the first recess,
**characterized in that** the first recess, comprises: a touch sensitive flat, planar surface (410) configured for sensing a first gesture thereon which activates the touch sensitive flat, planar surface, and for sensing a subsequent gesture thereon that invokes a predetermined action of the at least one touch sensitive control.

2. The ultrasound imaging system of claim 1, wherein the at least one touch sensitive control is circular in shape.

3. The ultrasound imaging system of claim 2, wherein the at least one touch sensitive control has a diameter in a range of 40 to 100 mm and a depth in a range of 0.1 to 1 mm.

4. The ultrasound imaging system of claim 1, wherein the at least one touch sensitive control is a touch sensitive trackball, and wherein a third gesture causes the touch sensitive control to move a graphical pointer displayed via the display in coordination with the third gesture.

5. An ultrasound imaging system (102), comprising:
a probe (104), including: a transducer array (106) with at least one transducer element (108);
a console (112), including: a controller (124) configured for controlling the at least one transducer element; and an echo processor;
a display monitor (122); and
a touch screen user interface (128), including: a touch panel (130) with a first major surface (402, 1402) and a first recess (408, 1408) in the first major surface; and at least one touch sensitive control (134) disposed in the first recess,
**characterized in that** the first recess, comprises: a second recess (1422) disposed therein, and a touch sensitive flat, actuating planar surface (1410), the second recess, comprising a touch sensitive flat, active planar surface (1424), wherein the touch sensitive flat, actuating planar surface is configured for sensing a first gesture thereon, which activates the touch sensitive flat, active planar surface (1424).

6. The ultrasound imaging system of claim 5, wherein the at least one touch sensitive control is elliptical in shape.

7. The ultrasound imaging system of claim 6, wherein the first recess has a first long axis in a range of 20 to 40 mm and a first depth in a range of 0.1 to 0.5 mm.

8. The ultrasound imaging system of claim 5, wherein the second recess is elliptical in shape.

9. The ultrasound imaging system of any of claims 5 to 8, wherein the second recess has a second long axis in a range of 20 to 30 mm and a second depth in a range of 0.1 to 0.5 mm.

10. The ultrasound imaging system of any of claims 5 to 9, wherein moving the first gesture from the touch sensitive flat, actuating planar surface to the touch sensitive flat, active planar surface invokes a predetermined action of the at least one touch sensitive control.

11. The ultrasound imaging system of claim 10, wherein the at least one touch sensitive control is toggle control, and wherein moving the first gesture in one direction from the touch sensitive flat, actuating planar surface to the touch sensitive flat, active planar surface increments a control parameter and moving the first gesture in an opposite direction from the touch sensitive flat, actuating planar surface to the touch sensitive flat, active planar surface decrements the control parameter.

## Patentansprüche

1. Ultraschall-Bildgebungssystem (102), umfassend:
eine Sonde (104), einschließend: eine Wandleranordnung (106) mit mindestens einem Wandlerelement (108);
eine Konsole (112), einschließend: eine Steuervorrichtung (124), die zum Steuern des mindestens einen Wandlerelements konfiguriert ist; und einen Echoprozessor;
einen Anzeigebildschirm (122); und
eine Berührungsbildschirm-Benutzeroberfläche (128), einschließend: ein Berührungspanel (130) mit einer ersten Hauptoberfläche (402, 1402) und einer ersten Aussparung (408, 1408) in der ersten Hauptoberfläche; und mindestens eine berührungsempfindliche Steuerung (134), die in der ersten Aussparung angeordnet ist,
**dadurch gekennzeichnet, dass** die erste Aussparung umfasst: eine berührungsempfindliche flache, planare Oberfläche (410), die zum Erfassen einer ersten Geste darauf konfiguriert ist, die die berührungsempfindliche flache, planare Oberfläche aktiviert, und zum Erfassen einer nachfolgenden Geste darauf, die eine vorbestimmte Aktion der mindestens einen berührungsempfindlichen Steuerung aufruft.

2. Ultraschall-Bildgebungssystem nach Anspruch 1, wobei die mindestens eine berührungsempfindliche Steuerung in kreisförmiger Form ist.

3. Ultraschall-Bildgebungssystem nach Anspruch 2, wobei die mindestens eine berührungsempfindliche Steuerung einen Durchmesser in einem Bereich von 40 bis 100 mm und eine Tiefe in einem Bereich von 0,1 bis 1 mm aufweist.

4. Ultraschall-Bildgebungssystem nach Anspruch 1, wobei die mindestens eine berührungsempfindliche Steuerung ein berührungsempfindlicher Trackball ist und wobei eine dritte Geste bewirkt, dass die berührungsempfindliche Steuerung einen über die Anzeige angezeigten grafischen Zeiger in Koordination mit der dritten Geste bewegt.

5. Ultraschall-Bildgebungssystem (102), umfassend:
eine Sonde (104), einschließend: eine Wandleranordnung (106) mit mindestens einem Wandlerelement (108);
eine Konsole (112), einschließend: eine Steuervorrichtung (124), die zur Steuerung des mindestens einen Wandlerelements konfiguriert ist; und einen Echoprozessor;
einen Anzeigebildschirm (122); und
eine Berührungsbildschirm-Benutzeroberfläche (128), einschließend: ein Berührungspanel (130) mit einer ersten Hauptoberfläche (402, 1402) und einer ersten Aussparung (408, 1408) in der ersten Hauptoberfläche; und mindestens eine berührungsempfindliche Steuerung (134), die in der ersten Aussparung angeordnet ist,
**dadurch gekennzeichnet, dass** die erste Aussparung umfasst: eine zweite Aussparung (1422), die darin angeordnet ist, und eine berührungsempfindliche, flache, betätigende, planare Oberfläche (1410), wobei die zweite Aussparung eine berührungsempfindliche, flache, betätigende, planare Oberfläche (1424) umfasst, wobei die berührungsempfindliche, flache, betätigende, planare Oberfläche konfiguriert ist, um eine erste Geste darauf zu erfassen, die die berührungsempfindliche, flache, aktive, planare Oberfläche (1424) aktiviert.

6. Ultraschall-Bildgebungssystem nach Anspruch 5, wobei die mindestens eine berührungsempfindliche Steuerung in elliptischer Form ist.

7. Ultraschall-Bildgebungssystem nach Anspruch 6, wobei die erste Aussparung eine erste Längsachse in einem Bereich von 20 bis 40 mm und eine erste Tiefe in einem Bereich von 0,1 bis 0,5 mm aufweist.

8. Ultraschall-Bildgebungssystem nach Anspruch 5, wobei die zweite Aussparung in elliptischer Form ist.

9. Ultraschall-Bildgebungssystem nach einem der Ansprüche 5 bis 8, wobei die zweite Aussparung eine zweite Längsachse in einem Bereich von 20 bis 30 mm und eine zweite Tiefe in einem Bereich von 0,1 bis 0,5 mm aufweist.

10. Ultraschall-Bildgebungssystem nach einem der Ansprüche 5 bis 9, wobei das Bewegen der ersten Geste von der berührungsempfindlichen flachen, betätigenden planaren Oberfläche zu der berührungsempfindlichen flachen, aktiven planaren Oberfläche eine vorbestimmte Aktion der mindestens einen berührungsempfindlichen Steuerung aufruft.

11. Ultraschall-Bildgebungssystem nach Anspruch 10, wobei die mindestens eine berührungsempfindliche Steuerung eine Umschaltsteuerung ist, und wobei das Bewegen der ersten Geste in einer Richtung von der berührungsempfindlichen flachen, betätigenden planaren Oberfläche zu der berührungsempfindlichen flachen, aktiven planaren Oberfläche einen Steuerparameter inkrementiert und das Bewegen der ersten Geste in einer entgegengesetzten Richtung von der berührungsempfindlichen flachen, betätigenden planaren Oberfläche zu der berührungsempfindlichen flachen, aktiven planaren Oberfläche den Steuerparameter dekrementiert.

## Revendications

1. Système d'échographie (102), comprenant :
une sonde (104), incluant: un réseau de transducteurs (106) comportant au moins un élément transducteur (108) ;
une console (112), incluant: un dispositif de commande (124) configuré pour commander le au moins un élément transducteur ; et un processeur d'écho ;
un moniteur d'affichage (122) ; et
une interface utilisateur à écran tactile (128), incluant : un panneau tactile (130) comportant une première surface principale (402, 1402) et un premier renfoncement (408, 1408) dans la première surface principale ; et au moins un dispositif de commande tactile (134) disposé dans le premier renfoncement,
**caractérisé en ce que** le premier renfoncement comprend : une surface plane, plate, tactile (410) configurée pour détecter un premier geste sur celle-ci qui active la surface plane, plate, tactile, et pour détecter un geste ultérieur sur celle-ci qui appelle une action prédéterminée du au moins un dispositif de commande tactile.

2. Système d'échographie selon la revendication 1, dans lequel le au moins un dispositif de commande tactile est de forme circulaire.

3. Système d'échographie selon la revendication 2, dans lequel le au moins un dispositif de commande tactile a un diamètre dans une plage de 40 à 100 mm et une profondeur dans une plage de 0,1 à 1 mm.

4. Système d'échographie selon la revendication 1, dans lequel le au moins un dispositif de commande tactile est une boule de commande tactile, et dans lequel un troisième geste amène le dispositif de commande tactile à déplacer un pointeur graphique affiché par l'intermédiaire du dispositif d'affichage en coordination avec le troisième geste.

5. Système d'échographie (102), comprenant :
une sonde (104), incluant: un réseau de transducteurs (106) comportant au moins un élément transducteur (108) ;
une console (112), incluant: un dispositif de commande (124) configuré pour commander le au moins un élément transducteur ; et un processeur d'écho ;
un moniteur d'affichage (122); et
une interface utilisateur à écran tactile (128), incluant : un panneau tactile (130) comportant une première surface principale (402, 1402) et un premier renfoncement (408, 1408) dans la première surface principale ; et au moins un dispositif de commande tactile (134) disposé dans le premier renfoncement,
**caractérisé en ce que** le premier renfoncement comprend : un second renfoncement (1422) disposé à l'intérieur de celui-ci, et une surface plane d'actionnement, plate, tactile (1410), le second renfoncement comprenant une surface plane active, plate, tactile (1424), dans lequel la surface plane d'actionnement, plate, tactile, est configurée pour détecter un premier geste sur celle-ci, qui active la surface plane active, plate, tactile (1424).

6. Système d'échographie selon la revendication 5, dans lequel le au moins un dispositif de commande tactile est de forme elliptique.

7. Système d'échographie selon la revendication 6, dans lequel le premier renfoncement a un premier grand axe dans une plage de 20 à 40 mm et une première profondeur dans une plage de 0,1 à 0,5 mm.

8. Système d'échographie selon la revendication 5, dans lequel le second renfoncement est de forme elliptique.

9. Système d'échographie selon l'une quelconque des revendications 5 à 8, dans lequel le second renfoncement a un second grand axe dans une plage de 20 à 30 mm et une seconde profondeur dans une plage de 0,1 à 0,5 mm.

10. Système d'échographie selon l'une quelconque des revendications 5 à 9, dans lequel un déplacement du premier geste de la surface plane d'actionnement, plate, tactile, à la surface plane active, plate, tactile, appelle une action prédéterminée du au moins un dispositif de commande tactile.

11. Système d'échographie selon la revendication 10, dans lequel le au moins un dispositif de commande tactile est un dispositif de commande à bascule, et dans lequel un déplacement du premier geste dans une direction de la surface plane d'actionnement, plate, tactile, à la surface plane active, plate, tactile, incrémente un paramètre de commande et un déplacement du premier geste dans une direction opposée de la surface plane d'actionnement, plate, tactile, à la surface plane active, plate, tactile, décrémente le paramètre de commande.
